# EUROPEAN PATENT SPECIFICATION

(11) **EP 1 576 153 B1**
(45) Date of publication and mention of the grant of the patent: **14.11.2007**
(21) Application number: 03814264.2
(22) Date of filing: 18.12.2003
(51) Int. Cl.: C12N 9/74, C12M 1/12

(54) **DEVICE AND PROCESS FOR THE PREPARATION OF AUTOLOGOUS THROMBIN SERUM**
VORRICHTUNG UND VERFAHREN ZUR HERSTELLUNG VON AUTOLOGEM THROMBINSERUM
DISPOSITIF ET PROCEDE DESTINES A LA PREPARATION DE SERUM AUTOLOGUE DE THROMBINE

(30) Priority: 23.12.2002 US 328419
(43) Date of publication of application: 21.09.2005
(73) Proprietor: Sorin Group USA, Inc., Arvada, CO 80004-3599 (US)
(72) Inventor: MCGINNIS, Daniel, Lakewood, CO 80226 (US); VOORHEES, Cherylyn, Arvada, CO 80005 (US); SMITH, Hubert, Denver, CO 80210 (US)
(74) Representative: Roberts, Mark Peter
(86) International application number: PCT/US2003/040804
(87) International publication number: WO 2004/058943

(56) References cited:
- WO-A1-00/74713
- US-A- 5 585 007
- US-B1- 6 274 090
- US-B1- 6 472 162
- HAISCH A ET AL: "PREPARATION OF A PURE AUTOLOGOUS BIODEGRADABLE FIBRIN MATRIX FOR TISSUE ENGINEERING" MEDICAL AND BIOLOGICAL ENGINEERING AND COMPUTING, PETER PEREGRINUS, STEVENAGE, GB, vol. 38, no. 6, November 2000 (2000-11), pages 686-689, XP001068027 ISSN: 0140-0118

## Description

The invention relates to a device and a process for increasing the concentration of cascade coagulation proteins in blood and, if desired, to activate the proteins. More particularly, the invention is used to convert prothrombin present in a plasma feedstock into thrombin.

Whole blood can be collected from a donor and processed into different products. Platelet rich plasma (PRP) is produced by separating plasma from red blood cells using a slow spin to prevent pelleting of the platelets. Platelet poor plasma (PPP) is produced by separating plasma from red blood cells using a high spin to pellet platelets with the red cells. Platelet poor plasma is the preferred starting material for thrombin.

Blood plasma proteins such as thrombin have many uses in medical, pharmaceutical, and industrial applications. For example, activated clotting cascade proteins are used to form bioengineered materials and to perform diagnostic testing on blood. Additionally, wound healing, hemostasis, and tissue adhesion products can be facilitated by the use of bio-engineered materials derived from whole blood. An example of a bioengineered material useful in wound healing is platelet gel. Platelet gel can be prepared by mixing a citrate-anticoagulated, platelet-rich plasma with calcium and thrombin. The solution is applied to a wound or reconstructive surgical site where it coagulates. Coagulation is the formation of a fibrin matrix in the platelet-rich plasma and occurs as a result of the cleavage of fibrinogen to fibrin due to the enzymatic action of thrombin and its calcium cofactor.

These blood-based bio-engineered materials all make use of the end stage of coagulation and all may utilize thrombin. However, finding a reliable and useable source of thrombin has proven to be a problem. Typically, the thrombin enzyme used to make the fibrin matrix is of bovine origin. Bovine thrombin has a potential to carry infectious agents and it can produce allergic reactions and coagulation disorders in humans, particularly upon re-exposure. Therefore, it is desirable to use autologous thrombin, i.e., thrombin obtained from a patient and then subsequently returned to the patient.

However, certain problems have been associated with the preparation and use of autologous thrombin which must be overcome in order for autologous thrombin to be viable for clinical use. For example, the process should be simple in its execution to eliminate error and should be capable of being performed using a disposable medical device to eliminate contamination. The process time for activation should be relatively short (e.g., ten to thirty minutes). Further, it is desirable for the activity of the thrombin serum so produced to induce coagulation quite rapidly, typically in less than five seconds, without causing an excessive dilution of the fibrinogen substrate to which it is added. In addition, the thrombin serum should be stable for the duration of a surgical procedure, which may take up to ten hours. WO 00/74713 discloses an apparatus to prepare thrombin from plasma, consisting of a reaction chamber connected to a syringe, and a filter between said chamber and syringe.

To date, there has been no simple or fast method that permits the collection of blood from a patient, preparation of autologous thrombin and/or platelet gel in a timely manner, and its return to the patient.

The invention provides a device and process for the preparation of thrombin serum from the blood of a single human donor. The thrombin serum may be used to form a bioengineered material useful for a variety of purposes including diagnostic blood testing, platelet gel for wound healing, and fibrin glues for hemostasis and tissue adhesion. In addition, thrombin alone can be used as a hemostasis agent. More particularly, the invention provides a device for the preparation of thrombin or other blood products from whole blood, plasma, or a plasma fraction according to claim 1.

It is to be understood that both the foregoing general description and the following detailed description are exemplary and explanatory and are intended to provide further explanation of the invention as claimed.

The invention will further be described, by way of example only, with reference to the accompanying drawings, in which:-
FIG. 1 illustrates a block diagram of a device of the invention; and
FIG. 2 illustrates a cut-away perspective view of a device of the invention.

The invention provides several advances in the art of the preparation of an autologous thrombin serum. The thrombin serum is reacted and formed in a syringe. In a preferred embodiment, the syringe is disposable.

In a device of the invention, whole blood, plasma, or a plasma fraction is exposed to components within a reaction chamber. These materials convert the whole blood, plasma, or the plasma fraction to the desired thrombin serum. After sufficient processing time, a serum is produced in either an activated or inactivated form. The serum contains clotting cascade proteins in concentrations higher than those occurring under normal physiological conditions. Active or inactive proteins may be conveniently removed from the reaction chamber and used in other processes and products.

A device of this invention facilitates conversion of prothrombin present in plasma feedstock into thrombin by a process that is simple and fast. The resulting concentrated autologous thrombin serum can be expressed through a filter of the device and can be used to catalyze the formation of unique bio-engineered materials having desired properties.

The invention provides a device for the preparation of thrombin or other blood products from whole blood, plasma, or a plasma fraction comprising: a reaction chamber having an inlet/outlet port; a filter located adjacent to the inlet/outlet port; a solid activating agent located inside the reaction chamber, the activating agent providing a surface for reaction; and a solid absorbent located inside the reaction chamber, wherein the device is a syringe having a distal region and comprising a plunger and a barrel and having the inlet/outlet port in the distal region of the syringe, the syringe forming a reaction chamber when the plunger is moved proximally out of the barrel; wherein the activating agent is selected from glass beads, diatomaceous earth, ceramics, kaolin, and combinations thereof; and wherein the resin excludes compounds having a molecular weight greater than 6000. In a preferred embodiment, the reaction chamber further comprises a source of calcium ions. In another preferred embodiment, the filter is located inside the reaction chamber. In a preferred embodiment, the total volume of the syringe is less than 100 ml.

In embodiments of the invention, the activating agent and the absorbent are located in discrete regions within the barrel of the syringe. The activating agent and the absorbent can be stratified disks oriented co-axially with the longitudinal axis of the barrel of the syringe. The activating agent can be adjacent to the filter and proximal of the filter, and the absorbent can be adjacent to the activating agent Alternatively, the activating agent and the absorbent may be mixed within the barrel.

In preferred embodiments, the filter is a porous plastic disk, preferably having a pore size of about 15 microns. The activating agent is selected from glass beads, diatomaceous earth, ceramics, kaolin, and combinations thereof. In another preferred embodiment, the activating agent comprises borosilicate glass beads. In preferred embodiments, the absorbent is a molecular exclusion gel that excludes compounds having a molecular weight greater than 6000. In a preferred embodiment, the source of calcium ions is CaCl₂. In another preferred embodiment, the source of calcium ions is an ion exchange resin comprising calcium ions. In a preferred embodiment, ethanol, plasma, and a source of calcium ions have been introduced into the device.

The invention provides a device for the preparation of thrombin or other blood products from whole blood, plasma, or a plasma fraction comprising: a reaction chamber having an inlet/outlet port; a filter located adjacent to the inlet/outlet port; a solid activating agent located inside the reaction chamber, the activating agent providing a surface for reaction; and an anionic, molecular exclusion, ion exchange resin located inside the reaction chamber, wherein the device is a syringe having a distal region and comprising a plunger and a barrel and having the inlet/outlet port in the distal region of the syringe, the syringe forming a reaction chamber when the plunger is moved proximally out of the barrel; wherein the activating agent is selected from glass beads, diatomaceous earth, ceramics, kaolin, and combinations thereof; and wherein the resin excludes compounds having a molecular weight greater than 6000. In a preferred embodiment, the resin comprises calcium ions. In another preferred embodiment, the filter is located inside the reaction chamber.

In a preferred embodiment, the total volume of the syringe is less than 100 ml.

In preferred embodiments, the filter is a porous plastic disk, preferably having a pore size of about 15 microns. The resin excludes compounds having a molecular weight greater than 6000. In a preferred embodiment, ethanol and plasma have been introduced into the device.

The device can be a rigid container. The thrombin can be removed from the rigid container by pumping from the container; by applying mechanical, pneumatic, or hydraulic force to a body which expresses thrombin from the container; or by centrifugation of the container to force the liquid from the packed bead bed.

The invention provides a method for making thrombin, comprising: introducing the plasma or the plasma fraction, ethanol, and a source of calcium ions into a device for the preparation of thrombin; allowing the plasma or the plasma fraction to react with the contents of the device for the preparation of thrombin to form thrombin; and removing the thrombin from the device for the preparation of thrombin; wherein the device for the preparation of thrombin comprises: a reaction chamber having an inlet/outlet port; a filter located adjacent to the inlet/outlet port; a solid activating agent located inside the reaction chamber, the activating agent providing a surface for reaction; and a solid absorbent located inside the reaction chamber, wherein the device is a syringe having a distal region and comprising a plunger and a barrel and having the inlet/outlet port in the distal region of the syringe, the syringe forming a reaction chamber when the plunger is moved proximally out of the barrel; wherein the activating agent is selected from glass beads, diatomaceous earth, ceramics, kaolin, and combinations thereof; and wherein the resin excludes compounds having a molecular weight greater than 6000. In a preferred embodiment, plasma is obtained from the patient and the plasma is platelet poor plasma. In another preferred embodiment, the filter is located inside the reaction chamber.

The invention provides a method for making thrombin, comprising: introducing the plasma or the plasma fraction, ethanol, and a source of calcium ions into a device for the preparation of thrombin; allowing the plasma or the plasma fraction to react with the contents of the device for the preparation of thrombin to form thrombin; and removing the thrombin from the device for the preparation of thrombin; wherein the device for the preparation of thrombin comprises: a reaction chamber having an inlet/outlet port; a filter located adjacent to the inlet/outlet port; a solid activating agent located inside the reaction chamber, the activating agent providing a surface for reaction; and an anionic, molecular exclusion, ion exchange resin located inside the reaction chamber, wherein the device is a syringe having a distal region and comprising a plunger and a barrel and having the inlet/outlet port in the distal region of the syringe, the syringe forming a reaction chamber when the plunger is moved proximally out of the barrel; wherein the activating agent is selected from glass beads, diatomaceous earth, ceramics, kaolin, and combinations thereof; and wherein the resin excludes compounds having a molecular weight greater than 6000.

In preferred embodiments, the invention provides autologous thrombin having an activity of greater than 50 International Units/ml (equivalent to 42 U.S. National Institutes of Health Units/ml), greater than 75 International Units/ml (equivalent to 63 U.S. National Institutes of Health Units/ml), or greater than 100 International Units/ml (equivalent to 84 U.S. National Institutes of Health Units/ml). In a preferred embodiment, the autologous thrombin was obtained from a human patient. The activity of the autologous thrombin can be determined by an in-vitro analysis using a chromogenic substrate measured spectrophotometrically. Test sample concentrations are determined by observing the rate of change in optical density and comparing these absorbances to that of a standard curve.

The methods could be used with any of the devices described above. In addition, other apparatuses could be used with the methods. The absorbent and the activating agent could be placed in a permeable bag (similar to a tea bag) and the permeable bag could be placed in a plasma, ethanol, and calcium source mixture. After reaction, the permeable bag can be removed and the thrombin rung out of the bag. An anionic, molecular exclusion, ion exchange resin can also be used in this type of apparatus.

The starting materials could be mixed in a beaker, allowed to react, and drained through filter paper to remove the thrombin. The filtering can be done by gravity or with a vacuum or pump assist.

In a preferred embodiment, a sample of blood is collected from a patient. The plasma is separated from other blood components by conventional methods. A syringe contains materials that result in the production of a thrombin serum when plasma (especially platelet poor plasma) is aspirated into it and mixed with these materials. A filter, integral with the syringe, retains these materials and undesired precipitates when the thrombin serum is expressed. These materials preferably include calcium chloride, an activating agent, ethanol, and a molecular exclusion gel.

Preferably, the reactive components and plasma fluid are contained in a syringe. A standard syringe with an ultra-sonically bonded integral filter located behind the nozzle provides a very low cost containment package and particle barrier. Instead of ultrasonic bonding, other joining mechanisms could be used, e.g., glues, RF welding, heat staking, interference only, etc. The syringe plunger can be manipulated to eject or pull fluids into the device reaction chamber. The integral filter prevents solid components from leaving the reaction chamber during operation, sterilization, shipping, and handling.

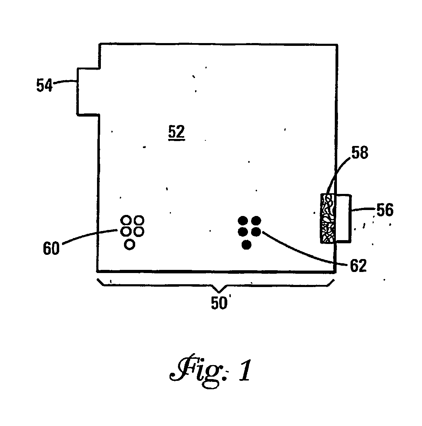

A device for the preparation of autologous thrombin is shown in FIG. 1. The device 50 comprises a reaction chamber 52 having an inlet port 54 and an outlet port 56. A filter 58 is located inside the reaction chamber 52 and adjacent to the outlet port 56. An activating agent 60 is located inside the reaction chamber. The activating agent 60 provides a surface for reaction. An absorbent 62 is located inside the reaction chamber.

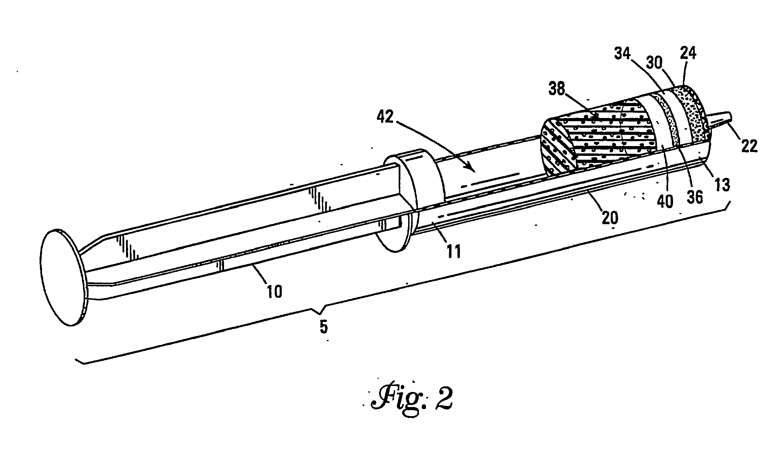

A device for the preparation of autologous thrombin is shown in cut-away view in FIG. 2. The device comprises a syringe 5 having a plunger 10 and barrel 20. In FIG. 2, plunger 10 is shown pulled out of barrel 20 at proximal end 11 thereof. At distal end 13 of the barrel is port 22. Adjacent port 22 is filter 24. In a preferred embodiment, filter 24 comprises a sintered porous plastic filter that is ultrasonically welded to the syringe providing a shunt free seal.

Barrel 20 is loaded with a glass bead activator 30, polyacrylamide gel 34 (this gel having a molecular exclusion of 1 to 6 kd, commercially available under the trade designation BIO-GEL P-6, available from Bio-Rad Laboratories, Hercules, CA), and powdered calcium chloride 36. When the operator desires to make a batch of autologous thrombin, plasma 38 is drawn into the syringe immediately followed by the addition of ethanol 40. An air bubble 42 is drawn into the syringe to allow sufficient space for mixing. The operator briefly agitates the syringe to mix and disperse the contents. A reaction period of approximately fifteen minutes is required to produce the thrombin serum. Alternatively, the calcium chloride could be included in the ethanol or it could be included in a separate solution drawn into the syringe.

The autologous thrombin serum can be stored in the syringe and dispensed as needed into desired aliquots to be mixed with other components. If the autologous thrombin serum is not needed immediately, it is preferred to chill the syringe and its contents. This is desirable because the thrombin tends to experience the onset of degradation after approximately one hour at room temperature.

The reaction chamber described above for the device of this invention can be included in a larger apparatus to provide protein processing steps that are used to produce other concentrated blood products such as: Factor XIII, Factor VIII or fibrinogen for fibrin sealant. Such an apparatus could automatically withdraw and depress the syringe plunger as required.

The materials included in a device used in making the autologous thrombin will now be described in more detail.

### Calcium Ions

Preferably, calcium ions are provided to reverse the action of the citrate anticoagulant present in plasma feedstock. A common practice for producing an autologous thrombin serum is to reverse the effect of the citrate anticoagulant by adding an amount of Ca⁺⁺ to the thrombin feedstock plasma sufficient not only to catalyze prothrombin, but also to include an excess such that when the thrombin/Ca⁺⁺ serum is combined with platelet-rich plasma (PRP) or fibrinogen concentrate, coagulation subsequently occurs. This "convenience" results in the addition to the feedstock of Ca⁺⁺ in a quantity that can be as much as 200x greater than its normal physiologic concentration. The high concentration of Ca⁺⁺ inhibits the activation of prothrombin to thrombin. A more effective formulation to produce an autologous thrombin serum uses no more Ca⁺⁺ than is necessary for restoration of the thrombin feedstock plasma to a normal physiologic concentration. It is recognized that Ca⁺⁺ is stoichiometrically combined with Factor VII at one step in the intrinsic coagulation pathway and is therefore no longer available for subsequent use as a catalytic cofactor. Furthermore, in practical use, an accommodation must be made for instances of over and under anticoagulation of the whole blood aliquot. Experimentation has shown that the optimal Ca⁺⁺ concentration range to be at or slightly below the normal physiological levels.

An ion exchange resin having a negative charge can hold calcium ions. These ions can subsequently be released onto blood proteins such that the coagulation cascade reaction can occur. This reverses the anticoagulation that the presence of citrate causes in plasma. Therefore, calcium ions attached to any negatively charged organic polymer resin may be used in the device of this invention. Suitable organic resins include resins of styrene, acrylic, styrene divinylbenzene, polyacrylamide, nylon, polyethylene, starch, cellulose, and the like. In addition, the negative charge present on the resin can also activate the blood proteins to further advance coagulation events.

Any nontoxic calcium salt may be used as a source for calcium ions in the device to reverse the citrate anticoagulant. Such salts may be organic or inorganic, as long as they can transfer Ca⁺⁺ to serum proteins. An example of a water insoluble material that transfers Ca⁺⁺ onto serum components is BIO-REX 70 ion exchange resin (Bio-Rad Laboratories, Hercules, CA) with calcium cation. Suitable organic calcium salts include calcium propionate and calcium acetate. Suitable inorganic salts include calcium hydroxide, calcium ammoniate, calcium carbide, calcium carbonate, calcium sulfate, calcium nitrate, and calcium pyrophosphate. Calcium chloride is the preferred source of calcium for the invention because it is very soluble in the serum, fast-acting, low cost, and does not significantly alter the pH of the plasma serum.

### Activating Agents

An activating agent is used to provide a surface for reaction. Preferably, the activating agent provides a negatively charged catalytic surface that simulates exposure of blood to vascular collagen. The intrinsic clotting cascade pathway is initiated by a process called contact activation, a surface and charge dependent phenomenon centered on the activation of Factor XII. Factor XII is highly susceptible to proteolysis because it is bound to surfaces via a charge interaction. The Factor XII precursor has areas of net positive charge that can interact with surfaces with a net negative charge. This charge binding induces conformational changes that enhance the molecule's ability to undergo activation by plasma kallikrein and Factor HK.

Materials commonly used for contact activation include borosilicate glass (i.e., hematology glass), diatomaceous earth, ceramics, and kaolin. Ion exchange resins may also be suitable. Ion exchange resins can provide three separate process functions in one single material: anionic activation of plasma proteins, a source of calcium to neutralize citrate, and molecular exclusion absorbance to remove water and low molecular weight fluids, thus, concentrating the high molecular weight constituents of the final serum.

It has been found that a glass bead with a diameter equal to or greater than the packing fraction void space of the molecular size exclusion gel, described further below, provides a structural support within these interstitial spaces that maintains porosity of the hydrated gel bed as pressure is applied to express the thrombin serum. Greater surface area of activator will increase the conversion reaction rate of prothrombin to thrombin. Lesser surface area of negatively charged activation material included in the device will slow down the reaction rate. Also, lower negative charge density present upon the activator surface will slow down the reaction rate. Conversely, greater negative charge density on the activator surface will increase the reaction rate.

In a preferred embodiment, the activating agent has a large surface area to enable a strong response that will decrease processing time and is of a size that is not detrimental to filtration of the thrombin serum. It has been demonstrated that a borosilicate glass with an anionic surface charge has these preferred features and can be used as an effective activating agent.

### Ethanol

Solvent fractionation reduces the solubility of coagulation proteins and decreases the dielectric constant of the serum that is processed in the device. Any slight reduction in the solubility of antithrombin III will cause it to precipitate and thereby increase the conversion reaction rate of prothrombin to thrombin. Although use of ethanol is preferred, any other relatively non-polar solvent such as methanol, propanol, acetone, or methyl isobutyl ketone will enhance the process and contribute to increased reaction rate.

Ethanol (i.e., Cohn-Oncley ethanol fractionation) is included for the purpose of reducing the solubility of certain coagulation proteins and for decreasing the dielectric constant of the plasma environment. Ethanol fractionation is used to precipitate and denature fibrinogen and antithrombin III. Removal of antithrombin III by ethanol fractionation circumvents its inhibitory function at three points in the intrinsic coagulation pathway for the catalysis of prothrombin to thrombin. Removal of this regulating factor in the intrinsic pathway decreases processing time. Also, ethanol is relatively non-toxic.

Ethanol fractionation is typically done at low temperature to prevent denaturation of proteins of interest. Denaturation of fibrinogen and antithrombin III is not an issue for the autologous thrombin process and, in fact, is a preferable outcome. The thrombin serum can be processed at room temperature to reduce processing time without degrading the activity of the serum.

Furthermore, the addition of ethanol decreases the dielectric constant of the plasma media. This facilitates interactions between the plasma proteins and the surrounding ionic environment to reduce processing time.

### Absorbent

An absorbent is included in the reaction chamber of the device to absorb water. Any material that absorbs water and which does not denature the thrombin can be used as an absorbent. A molecular exclusion gel is a preferred absorbent. Molecular exclusion gel beads absorb water and thus concentrate the coagulation proteins in the plasma feedstock. This concentration step produces a thrombin serum with an activity level (National Institute of Health Units/ml) greater than achievable under normal physiologic levels. Materials suitable for this use are typically desiccated and/or hydrophilic chromatographic gels (e.g., agarose, dextrose, silica, or polyacrylamide). In preferred embodiments, the molecular exclusion gel is a polyacrylamide gel that excludes compounds having a molecular weight greater than 6000, 20,000, or 30,000. It is necessary, however, to maintain an optimum aqueous processing environment simultaneously with the concentration of the thrombin serum. Enzymatic processes are sensitive to substrate concentration, pH, and ionic concentration. Efficient plasma fractionation requires that ethanol be maintained at a concentration that maximizes precipitation of fibrinogen and antithrombin III and minimizes its impact on thrombin activation. Removal of water from the plasma to concentrate the prothrombin substrate will unfavorably increase ionic and ethanol concentrations. Therefore, the absorbent used to remove the water should be selected so that it preserves and maintains an optimum aqueous environment for these proteins.

The intrinsic coagulation pathway uses a four-step enzyme/substrate cascade amplification topology to catalyze prothrombin. Enzyme catalyzed reaction rates are maximized when the substrate concentration saturates the available enzymatic factor. Using a desiccant to absorb water from the plasma feedstock will concentrate the coagulation factor substrates, maximize the reaction rates, and minimize processing time.

An anionic, molecular exclusion, ion exchange resin can perform three process functions when calcium ions are the cationic charge balance species: molecular exclusion to remove water and concentrate the serum proteins, a source of calcium to reverse anticoagulation, and negative charge to activate the clotting cascade.

In a preferred embodiment, a chromatographic gel with the appropriate desalting and molecular-weight size-exclusion characteristics is used (such as a BIO-GEL P-6 gel having 1000-6000 M.W. exclusion limit range) because ionic and ethanol concentrations can be maintained at levels that are optimum for the aqueous processing environment concurrently with the concentration of the thrombin serum. For example, a polyacrylamide gel with molecular exclusion of 1 to 6 kd (such as BIO-GEL P-6) removes water in sufficient quantities to effectively increase serum protein concentration. A greater quantity of gel material included within the device will remove more water and thus increase the prothrombin to thrombin conversion reaction rate, as well as yield a serum with higher thrombin concentration than that which is produced by a device that includes lower quantities of molecular exclusion gel.

A device of this invention also provides for an increase in thrombin serum lifetime. Thrombin autocatalyses at a rate proportional to its concentration. Chilling the thrombin serum to a temperature of 1.5°C to 4.5°C can minimize this degradation but cannot reverse the loss in thrombin activity (U/ml). The hydration characteristic of the chromatographic gel can be described by a time constant of approximately 0.5 to 1 hour, with essentially complete hydration of the gel occurring in a period of four time constants. A chilled thrombin serum when maintained in contact with the gel will become increasingly concentrated and therefore will not see a loss in activity over a period of three to four time constants.

### Device Integral Filter

The device reaction chamber contains particulate materials that interact with plasma to facilitate chemical reaction. These solid materials should not transfer and contaminate the final serum. Therefore, the device should have a retention barrier in place such that fluid can be aspirated, as well as ejected with ease. A single layer porous plastic disk can be affixed to the orifice of the reagent containment chamber such as by ultra-sonic welding. A sandwiched composite filter composed of several layers of dissimilar materials with different filtration performance characteristics can be welded into place with a one step process. The composite filter assembly can include an o-ring structure to provide extra weld melt plastic material to assist with the integrity and sealing capability of the filter. The o-ring can have a cross member structure to provide additional support to help retain thin filter layers placed underneath. The preferred filter, however, is a single disk of sintered porous polyethylene having a pore size of 15 microns (such as commercially available under the trade designation POREX, available from Porex Corporation, Fairburn, GA). This filter is preferred because it is low in cost and easy to assemble.

### Example

A standard 30 ml polypropylene syringe was prepared by ultrasonic welding of a 1/8-inch (0.32 cm) thick, 15 µm pore-size, sintered-porous polyethylene plastic filter to the syringe vertex. The syringe was loaded with 8 gm of 80 µm diameter borosilicate glass beads, 2.2 gm of polyacrylamide gel (BIO-GEL P-6, available from Bio-Rad Laboratories, Hercules, CA), and 0.20 mM of CaCl₂ powder.

Twelve ml of citrate anticoagulated platelet poor plasma (PPP), drawn 24 hours previously, was aspirated into the syringe. Immediately thereafter, 2.5 ml of 195 proof ethanol was aspirated into the syringe. A mixing bubble was provided and the device was briefly hand agitated to mix and disperse the contents. The syringe was placed vertically on the plunger end cap and allowed to incubate for 14 minutes. At approximately 12 to 13 minutes the viscosity of the plasma mixture was observed to increase significantly. At 14 minutes, 5.5 ml of an autologous thrombin serum was expressed from the device and chilled to approximately 4.5°C.

The thrombin serum was assayed and found to have an activity of 93 National Institutes of Health Units/ml (111 International Units/ml). A 10:1 volume ratio platelet rich plasma (PRP) to thrombin serum platelet gel was observed to coagulate in less than 5 seconds. A 40:1 volume ratio PRP to thrombin serum platelet gel was observed to coagulate in approximately 35 seconds.

This example illustrates a convenient method to generate autologous thrombin having activity that is useful for clinical applications such as forming platelet gel. The gel does not contain any bovine thrombin, which is commonly used as a gel catalyst. This is desirable because the patient receiving the gel is not exposed to potential hazards associated with bovine thrombin such as mad cow disease or immune reaction. The process time of fourteen minutes is fast enough to accommodate the time frame of a clinical procedure using platelet gel or other clinical procedures. The device is simple to use because it does not require any complex equipment to facilitate the conversion reaction. The device is easy to operate because it only takes three process steps: (1) fill the syringe with ethanol and plasma, (2) shake contents, and (3) let contents react at room temperature for fourteen minutes.

In a preferred embodiment, the reaction chamber of the thrombin device contains two dry powder components: borosilicate glass beads with diameters of 80 to 120 micron, and polyacrylamide gel beads with diameters of 40 to 90 micron. Ethylene oxide gas, commonly used to sterilize medical devices, does not effectively kill bacteria existing between and on the surfaces of the device's powder components. Ethylene oxide only kills bacteria in the presence of water. This sterilization process utilizes a primary conditioning steam injection step before the introduction of ethylene gas. The steam condenses and re-hydrates gel beads contained within the device. The resulting hydrated gel layer is impervious to ethylene oxide gas penetration and forms a protective layer that encapsulates interstitial bacteria so that it cannot be destroyed. Therefore, another method is used to sterilize the device.

Electron beam and gamma radiation are two types of energy that are commonly used to sterilize medical devices. Both of these energy forms kill by breaking chemical bonds essential to life material within bacteria. Electron beam radiation used in sterilization processes is approximately 1000 times more powerful than gamma energy, and also, has a negative charge. Gamma energy consists of photons having no charge.

The thrombin device's glass beads have an anionic surface charge that stimulates intrinsic coagulation of blood plasma (in the presence of calcium). Electron beam radiation denatures the anionic surface charge of glass such that coagulation of plasma held in the device reaction chamber does not occur in a timely manner. Low energy gamma radiation is sufficient to effectively kill bacteria present in the thrombin device (within acceptable bio-burden limits) yet not denature the anionic surface charge of glass. However, gamma radiation alters the device's gel material by de-crosslinking the polymer network. This reduces the gel's ability to absorb blood plasma water in the reaction chamber. Therefore, the quantity of gel contained within the device must be increased to compensate for post-gamma sterilization gel absorbency reduction.

A lower concentration of pro-thrombin, resulting from the presence of more water in the device reaction chamber, results in a longer time required to convert pro-thrombin to thrombin, as well as, a more dilute final serum. Therefore, the optimal gamma sterilized device design includes increasing gel bead weight from an un-sterilized specification of 2.10+/- 0.10 grams to 2.25 +/- 0.10 grams sterilized.

Accordingly, the invention provides a method for compensating for the degradation in the absorbency of a molecular exclusion gel due to low energy gamma radiation sterilization comprising: determining the absorbency of the unsterilized and sterilized molecular exclusion gel; and adding an additional amount of molecular exclusion gel to compensate for the degradation in absorbency. In a preferred embodiment, the molecular exclusion gel is a polyacrylamide gel.

The above description and the drawings are provided for the purpose of describing embodiments of the invention and are not intended to limit the scope of the invention in any way. It will be apparent to those skilled in the art that various modifications and variations can be made without departing from the spirit or scope of the invention. Thus, it is intended that the present invention cover the modifications and variations of this invention provided they come within the scope of the appended claims.

## Claims

1. A device for the preparation of thrombin or other blood products from whole blood, plasma, or a plasma fraction comprising:
a reaction chamber having an inlet/outlet port;
a filter located adjacent to the inlet/outlet port;
a activating agent located inside the reaction chamber, the activating agent providing a surface for reaction; and
a absorbent located inside the reaction chamber,
wherein the device is a syringe having a distal region and comprising a plunger and a barrel and having the inlet/outlet port in the distal region of the syringe, the syringe forming a reaction chamber when the plunger is moved proximally out of the barrel;
wherein the activating agent is selected from glass beads, diatomaceous earth, ceramics, kaolin, and combinations thereof; and
wherein the absorbent is a molecular exclusion gel that excludes compounds having a molecular weight greater than 6000.

2. A device of claim 1, wherein the reaction chamber further comprises a source of calcium ions.

3. A device of claim 1, wherein the activating agent and the absorbent are located in discrete regions within the barrel of the syringe.

4. A device of claim 3, wherein the activating agent and the absorbent are stratified disks oriented co-axially with the longitudinal axis of the barrel of the syringe.

5. A device of claim 4, wherein the activating agent is adjacent to the filter and proximal of the filter, and the absorbent is adjacent to the activating agent.

6. A device of claim 1, wherein the activating agent and the absorbent are mixed together.

7. A device for the preparation of thrombin or other blood products from whole blood, plasma, or a plasma fraction comprising:
a reaction chamber having an inlet/outlet port;
a filter located adjacent to the inlet/outlet port;
a activating agent located inside the reaction chamber, the activating agent providing a surface for reaction; and
an anionic, molecular exclusion, ion exchange resin located inside the reaction chamber,
wherein the device is a syringe having a distal region and comprising a plunger and a barrel and having the inlet/outlet port in the distal region of the syringe, the syringe forming a reaction chamber when the plunger is moved proximally out of the barrel;
wherein the activating agent is selected from glass beads, diatomaceous earth, ceramics, kaolin, and combinations thereof; and
wherein the resin excludes compounds having a molecular weight greater than 6000.

8. A device of claim 1 or 7, wherein the filter is located inside the reaction chamber.

9. A device of claim 1 or 7, wherein the filter is a porous plastic disk.

10. A device of claim 1 or 7, wherein the filter is a disk of porous plastic having a pore size of about 15 microns.

11. A device of claim 1, wherein ethanol, plasma, and a source of calcium ions have been introduced into the device.

12. A device of claim 7, wherein ethanol and plasma have been introduced into the device.

13. A method for making thrombin, comprising:
introducing a plasma or a plasma fraction, ethanol, and a source of calcium ions into a device for the preparation of thrombin;
allowing the plasma or the plasma fraction to react with the contents of the device for the preparation of thrombin to form thrombin; and
removing the thrombin from the device for the preparation of thrombin;
wherein the device for the preparation of thrombin comprises:
a reaction chamber having an inlet/outlet port;
a filter located adjacent to the inlet/outlet port;
a activating agent located inside the reaction chamber, the activating agent providing a surface for reaction; and
a absorbent located inside the reaction chamber,
wherein the device is a syringe having a distal region and comprising a plunger and a barrel and having the inlet/outlet port in the distal region of the syringe, the syringe forming a reaction chamber when the plunger is moved proximally out of the barrel;
wherein the activating agent is selected from glass beads, diatomaceous earth, ceramics, kaolin, and combinations thereof; and
wherein the absorbent is a molecular exclusion gel that excludes compounds having a molecular weight greater than 6000.

14. A method for making thrombin, comprising:
introducing a plasma or a plasma fraction, ethanol, and a source of calcium ions into a device for the preparation of thrombin;
allowing the plasma or the plasma fraction to react with the contents of the device for the preparation of thrombin to form thrombin; and
removing the thrombin from the device for the preparation of thrombin;
wherein the device for the preparation of thrombin comprises:
a reaction chamber having an inlet/outlet port;
a filter located adjacent to the inlet/outlet port;
a activating agent located inside the reaction chamber, the activating agent providing a surface for reaction; and
an anionic, molecular exclusion, ion exchange resin located inside the reaction chamber,
wherein the device is a syringe having a distal region and comprising a plunger and a barrel and having the inlet/outlet port in the distal region of the syringe, the syringe forming a reaction chamber when the plunger is moved proximally out of the barrel;
wherein the activating agent is selected from glass beads, diatomaceous earth, ceramics, kaolin, and combinations thereof; and
wherein the resin excludes compounds having a molecular weight greater than 6000.

15. A method of claim 13 or 14, wherein the filter is located inside the reaction chamber.

16. A device of claim 1 or 7 or a method of claim 13 or 14, wherein the activating agent comprises glass beads.

17. A device of claim 1 or 7 or a method of claim 13 or 14, wherein the activating agent comprises borosilicate glass beads.

18. A device of claim 1 or a method of claim 13, wherein the absorbent is a molecular exclusion gel that excludes compounds having a molecular weight greater than 20,000.

19. A device of claim 1 or a method of claim 13, wherein the absorbent is a molecular exclusion gel that excludes compounds having a molecular weight greater than 30,000.

20. A device of claim 2 or 11 or a method of claim 13 or 14, wherein the source of calcium ions is CaCl₂.

21. A device of claim 2 or a method of claim 13, wherein the source of calcium ions is an ion exchange resin comprising calcium ions.

22. A method of claim 13 or 14, wherein the plasma is platelet poor plasma.

23. A device of claim 7, wherein the resin comprises calcium ions.

24. A device of claim 7 or a method of claim 14, wherein the resin excludes compounds having a molecular weight greater than 20,000.

25. A device of claim 7 or a method of claim 14, wherein the resin excludes compounds having a molecular weight greater than 30,000.

## Patentansprüche

1. Vorrichtung für die Präparierung von Thrombin oder anderen Blutprodukten aus Vollblut, Plasma oder einer Plasmafraktion, umfassend:
eine Reaktionskammer mit einer Einlass/Ablassöffnung;
einen Filter, der angrenzend an die Einlass/Ablassöffnung lokalisiert ist;
ein Aktivierungsmittel, das innerhalb der Reaktionskammer lokalisiert ist, wobei das Aktivierungsmittel eine Oberfläche für die Reaktion bereitstellt; und ein Absorbens, das innerhalb der Reaktionskammer lokalisiert ist,
wobei die Vorrichtung eine Spritze ist, die eine distale Region aufweist und einen Spritzenkolben und ein Spritzengehäuse umfasst und die die Einlass/Ablassöffnung in der distalen Region der Spritze aufweist, wobei die Spritze eine Reaktionskammer bildet, wenn der Spritzenkolben proximal aus dem Spritzengehäuse gezogen ist;
wobei das Aktivierungsmittel ausgewählt ist aus Glaskügelchen, Diatomeenerde, Keramik, Kaolin und Kombinationen davon; und
wobei das Absorbens ein Molekül-ausschließendes Gel ist, das Verbindungen mit einem Molekulargewicht von größer als 6000 ausschließt.

2. Vorrichtung nach Anspruch 1, wobei die Reaktionskammer außerdem eine Quelle von Calciumionen umfasst.

3. Vorrichtung nach Anspruch 1, wobei das Aktivierungsmittel und das Absorbens in getrennten Regionen innerhalb des Gehäuses der Spritze lokalisiert sind.

4. Vorrichtung nach Anspruch 3, wobei das Aktivierungsmittel und das Absorbens aufeinander geschichtete Scheiben sind, die koaxial mit der Längsachse des Gehäuses der Spritze orientiert sind.

5. Vorrichtung nach Anspruch 4, worin sich das Aktivierungsmittel angrenzend an den Filter und proximal zu dem Filter befindet und sich das Absorbens angrenzend an das Aktivierungsmittel befindet.

6. Vorrichtung nach Anspruch 1, worin das Aktivierungsmittel und das Absorbens miteinander vermischt sind.

7. Vorrichtung für die Präparierung von Thrombin oder anderen Blutprodukten aus Vollblut, Plasma oder einer Plasmafraktion, umfassend:
eine Reaktionskammer mit einer Einlass/Ablassöffnung;
einen Filter, der angrenzend an die Einlass/Ablassöffnung lokalisiert ist;
ein Aktivierungsmittel, das innerhalb der Reaktionskammer lokalisiert ist, wobei das Aktivierungsmittel eine Oberfläche für die Reaktion bereitstellt; und
ein anionisches molekülausschließendes lonenaustauschharz, das innerhalb der Reaktionskammer lokalisiert ist,
wobei die Vorrichtung eine Spritze ist, die eine distale Region aufweist und einen Spritzenkolben und ein Spritzengehäuse umfasst und die die Einlass/Ablassöffnung in der distalen Region der Spritze aufweist, wobei die Spritze eine Reaktionskammer bildet, wenn der Spritzenkolben proximal aus dem Spritzengehäuse gezogen ist;
wobei das Aktivierungsmittel ausgewählt ist aus Glaskügelchen, Diatomeenerde, Keramik, Kaolin und Kombinationen davon; und
wobei das Harz Verbindungen mit einem Molekulargewicht von größer als 6000 ausschließt.

8. Vorrichtung nach Anspruch 1 oder 7, wobei der Filter innerhalb der Reaktionskammer lokalisiert ist.

9. Vorrichtung nach Anspruch 1 oder 7, wobei der Filter eine poröse Kunststoffscheibe ist.

10. Vorrichtung nach Anspruch 1 oder 7, wobei der Filter eine Scheibe aus porösem Kunststoff mit einer Porengröße von etwa 15 Mikronen ist.

11. Vorrichtung nach Anspruch 1, wobei Ethanol, Plasma und eine Quelle von Calciumionen in die Vorrichtung eingeführt worden sind.

12. Vorrichtung nach Anspruch 7, wobei Ethanol und Plasma in die Vorrichtung eingeführt worden sind.

13. Methode zur Bildung von Thrombin, umfassend:
Einführen eines Plasmas oder einer Plasmafraktion, Ethanol und einer Quelle von Calciumionen in eine Vorrichtung für die Präparierung von Thrombin;
Reagierenlassen des Plasmas oder der Plasmafraktion mit den Inhaltsstoffen der Vorrichtung für die Präparierung von Thrombin zur Bildung von Thrombin; und
Entfernen des Thrombins aus dem Vorrichtung für die Präparierung von Thrombin;
wobei die Vorrichtung für die Präparierung von Thrombin umfasst:
eine Reaktionskammer mit einer Einlass/Ablassöffnung;
einen Filter, der angrenzend an die Einlass/Ablassöffnung lokalisiert ist;
ein Aktivierungsmittel, das innerhalb der Reaktionskammer lokalisiert ist, wobei das Aktivierungsmittel eine Oberfläche für die Reaktion bereitstellt; und
ein Absorbens, das innerhalb der Reaktionskammer lokalisiert ist,
wobei die Vorrichtung eine Spritze ist, die eine distale Region aufweist und einen Spritzenkolben und ein Spritzengehäuse umfasst und die die Einlass/Ablassöffnung in der distalen Region der Spritze aufweist, wobei die Spritze eine Reaktionskammer bildet, wenn der Spritzenkolben proximal aus dem Spritzengehäuse gezogen ist;
wobei das Aktivierungsmittel ausgewählt ist aus Glaskügelchen, Diatomeenerde, Keramik, Kaolin und Kombinationen davon; und
wobei das Absorbens ein Molekül-ausschließendes Gel ist, das Verbindungen mit einem Molekulargewicht von größer als 6000 ausschließt.

14. Methode zur Bildung von Thrombin, umfassend:
Einführen eines Plasmas oder einer Plasmafraktion, Ethanol und einer Quelle von Calciumionen in eine Vorrichtung für die Präparierung von Thrombin;
Reagierenlassen des Plasmas oder der Plasmafraktion mit den Inhaltsstoffen der Vorrichtung für die Präparierung von Thrombin zur Bildung von Thrombin; und
Entfernen des Thrombins aus der Vorrichtung für die Präparierung von Thrombin;
wobei die Vorrichtung für die Präparierung von Thrombin umfasst:
eine Reaktionskammer mit einer Einlass/Ablassöffnung;
einen Filter, der angrenzend an die Einlass/Ablassöffnung lokalisiert ist;
ein Aktivierungsmittel, das innerhalb der Reaktionskammer lokalisiert ist, wobei das Aktivierungsmittel eine Oberfläche für die Reaktion bereitstellt; und
ein anionisches molekülausschließendes lonenaustauschharz, das innerhalb der Reaktionskammer lokalisiert ist,
wobei die Vorrichtung eine Spritze ist, die eine distale Region aufweist und einen Spritzenkolben und ein Spritzengehäuse umfasst und die die Einlass/Ablassöffnung in der distalen Region der Spritze aufweist, wobei die Spritze eine Reaktionskammer bildet, wenn der Spritzenkolben proximal aus dem Spritzengehäuse gezogen ist;
wobei das Aktivierungsmittel ausgewählt ist aus Glaskügelchen, Diatomeenerde, Keramik, Kaolin und Kombinationen davon; und
wobei das Harz Verbindungen mit einem Molekulargewicht von größer als 6000 ausschließt.

15. Methode nach Anspruch 13 oder 14, wobei der Filter innerhalb der Reaktionskammer lokalisiert ist.

16. Vorrichtung nach Anspruch 1 oder 7 oder Methode nach Anspruch 13 oder 14, wobei das Aktivierungsmittel Giaskügeichen umfasst.

17. Vorrichtung nach Anspruch 1 oder 7 oder Methode nach Anspruch 13 oder 14, wobei das Aktivierungsmittel Borsilikat-Glaskügelchen umfasst.

18. Vorrichtung nach Anspruch 1 oder Methode nach Anspruch 13, wobei das Absorbens ein Molekül-ausschließendes Gel ist, das Verbindungen mit einem Molekulargewicht von größer als 20.000 ausschließt.

19. Vorrichtung nach Anspruch 1 oder Methode nach Anspruch 13, wobei das Absorbens ein Molekül-ausschließendes Gel ist, das Verbindungen mit einem Molekulargewicht von größer als 30.000 ausschließt.

20. Vorrichtung nach Anspruch 2 oder 11 oder Methode nach Anspruch 13 oder 14, wobei die Quelle der Calciumionen CaCl₂ ist.

21. Vorrichtung nach Anspruch 2 oder Methode nach Anspruch 13, wobei die Quelle der Calciumionen ein lonenaustauschharz, umfassend Calciumionen, ist.

22. Methode nach Anspruch 13 oder 14, wobei das Plasma ein Blutplättchen-armes Plasma ist.

23. Vorrichtung nach Anspruch 7, wobei das Harz Calciumionen umfasst.

24. Vorrichtung nach Anspruch 7 oder Methode nach Anspruch 14, wobei das Harz Verbindungen mit einem Molekulargewicht von größer als 20.000 ausschließt.

25. Vorrichtung nach Anspruch 7 oder Methode nach Anspruch 14, wobei das Harz Verbindungen mit einem Molekulargewicht von größer als 30.000 ausschließt.

## Revendications

1. Dispositif pour la préparation de produits de thrombine ou d'autres produits sanguins à partir de sang entier, de plasma, ou d'une fraction de plasma, comprenant :
une chambre de réaction ayant un orifice d'entrée/sortie ;
un filtre situé adjacent à l'orifice d'entrée/sortie ;
un agent d'activation situé à l'intérieur de la chambre de réaction, l'agent d'activation offrant une surface pour la réaction ; et
un absorbant situé à l'intérieur de la chambre de réaction,
dans lequel le dispositif est une seringue ayant une région distale et comprenant un plongeur et un cylindre et ayant l'orifice d'entrée/sortie dans la région distale de la seringue, la seringue formant une chambre de réaction lorsque le plongeur est déplacé de manière proximale hors du cylindre ;
dans lequel l'agent d'activation est choisi parmi des perles de verre, de la terre à diatomée, de la céramique, du kaolin, et des combinaisons de ceux-ci ; et
dans lequel l'absorbant est un gel d'exclusion moléculaire qui exclut des composés ayant un poids moléculaire supérieur à 6000.

2. Dispositif selon la revendication 1, dans lequel la chambre de réaction comprend en outre une source d'ions calcium.

3. Dispositif selon la revendication 1, dans lequel l'agent d'activation et l'absorbant sont situés dans des régions discrètes à l'intérieur du cylindre de la seringue.

4. Dispositif selon la revendication 3, dans lequel l'agent d'activation et l'absorbant sont des disques stratifiés orientés de manière coaxiale avec l'axe longitudinal du cylindre de la seringue.

5. Dispositif selon la revendication 4, dans lequel l'agent d'activation est adjacent au filtre et proximal du filtre, et l'absorbant est adjacent à l'agent d'activation.

6. Dispositif selon la revendication 1, dans lequel l'agent d'activation et l'absorbant sont mélangés ensemble.

7. Dispositif pour la préparation de produits de thrombine ou d'autres produits sanguins à partir de sang entier, de plasma, ou d'une fraction de plasma, comprenant :
une chambre de réaction ayant un orifice d'entrée/sortie ;
un filtre situé adjacent à l'orifice d'entrée/sortie ;
un agent d'activation situé à l'intérieur de la chambre de réaction, l'agent d'activation offrant une surface pour la réaction ; et
une résine échangeuse d'ions, anionique, à exclusion moléculaire, située à l'intérieur de la chambre de réaction,
dans lequel le dispositif est une seringue ayant une région distale et comprenant un plongeur et un cylindre et ayant l'orifice d'entrée/sortie dans la région distale de la seringue, la seringue formant une chambre de réaction lorsque le plongeur est déplacé de manière proximale hors du cylindre ;
dans lequel l'agent d'activation est choisi parmi des perles de verre, de la terre à diatomées, des céramiques, du kaolin, et des combinaisons de ceux-ci ; et
dans lequel la résine exclut des composés ayant un poids moléculaire supérieur à 6000.

8. Dispositif selon la revendication 1 ou 7, dans lequel le filtre est situé à l'intérieur de la chambre de réaction.

9. Dispositif selon la revendication 1 ou 7, dans lequel le filtre est un disque de matière plastique poreuse.

10. Dispositif selon la revendication 1 ou 7, dans lequel le filtre est un disque de matière plastique poreuse ayant une taille de pore d'environ 15 microns.

11. Dispositif selon la revendication 1, dans lequel de l'éthanol, du plasma, et une source d'ions calcium ont été introduits dans le dispositif.

12. Dispositif selon la revendication 7, dans lequel de l'éthanol et du plasma ont été introduits dans le dispositif.

13. Procédé pour fabriquer de la thrombine, comprenant :
l'introduction d'un plasma ou d'une fraction de plasma, d'éthanol, et d'une source d'ions calcium dans un dispositif de préparation de thrombine ;
la mise en réaction du plasma ou de la fraction de plasma avec le contenu du dispositif de préparation de thrombine pour former de la thrombine ; et
le retrait de la thrombine depuis le dispositif de préparation de thrombine ;
dans lequel le dispositif de préparation de thrombine comprend:
une chambre de réaction ayant un orifice d'entrée/sortie ;
un filtre situé adjacent à l'orifice d'entrée/sortie ;
un agent d'activation situé à l'intérieur de la chambre de réaction, l'agent d'activation offrant une surface pour la réaction ; et
un absorbant situé à l'intérieur de la chambre de réaction,
dans lequel le dispositif est une seringue ayant une région distale et comprenant un plongeur et un cylindre et ayant l'orifice d'entrée/sortie dans la région distale de la seringue, la seringue formant une chambre de réaction lorsque le plongeur est déplacé de manière proximale hors du cylindre ;
dans lequel l'agent d'activation est choisi parmi des perles de verre, de la terre à diatomées, des céramiques, du kaolin, et des combinaisons de ceux-ci; et
dans lequel l'absorbant est un gel d'exclusion moléculaire qui exclut des composés ayant un poids moléculaire supérieur à 6000.

14. Procédé pour fabriquer de la thrombine, comprenant :
l'introduction d'un plasma ou d'une fraction de plasma, d'éthanol, et d'une source d'ions calcium dans un dispositif de préparation de thrombine ;
la mise en réaction du plasma ou de la fraction de plasma avec le contenu du dispositif de préparation de thrombine pour former de la thrombine ; et
le retrait de la thrombine depuis le dispositif de préparation de thrombine;
dans lequel le dispositif de préparation de thrombine comprend:
une chambre de réaction ayant un orifice d'entrée/sortie ;
un filtre situé adjacent à l'orifice d'entrée/sortie ;
un agent d'activation situé à l'intérieur de la chambre de réaction, l'agent d'activation offrant une surface pour la réaction ; et
une résine échangeuse d'ions, anionique, à exclusion moléculaire, située à l'intérieur de la chambre de réaction,
dans lequel le dispositif est une seringue ayant une région distale et comportant un plongeur et un cylindre et ayant l'orifice d'entrée/sortie dans la région distale de la seringue, la seringue formant une chambre de réaction lorsque le plongeur est déplacé de manière proximale hors du cylindre ;
dans lequel l'agent d'activation est choisi parmi des perles de verre, de la terre à diatomées, des céramiques, du kaolin, et des combinaisons de ceux-ci ; et
dans lequel la résine exclut des composés ayant un poids moléculaire supérieur à 6000.

15. Procédé selon la revendication 13 ou 14, dans lequel le filtre est situé à l'intérieur de la chambre de réaction.

16. Dispositif selon la revendication 1 ou 7 ou procédé selon la revendication 13 ou 14, dans lequel l'agent d'activation comprend des perles de verre.

17. Dispositif selon la revendication 1 ou 7 ou procédé selon la revendication 13 ou 14, dans lequel l'agent d'activation comprend des perles de verre borosilicaté.

18. Dispositif selon la revendication 1 ou procédé selon la revendication 13, dans lequel l'absorbant est un gel d'exclusion moléculaire qui exclut des composés ayant un poids moléculaire supérieur à 20 000.

19. Dispositif selon la revendication 1 ou procédé selon la revendication 13, dans lequel l'absorbant est un gel d'exclusion moléculaire qui exclut des composés ayant un poids moléculaire supérieur à 30 000.

20. Dispositif selon la revendication 2 ou 11 ou procédé selon la revendication 13 ou 14, dans lequel la source d'ions calcium est CaCl₂.

21. Dispositif selon la revendication 2 ou procédé selon la revendication 13, dans lequel la source d'ions calcium est une résine échangeuse d'ions comprenant des ions calcium.

22. Procédé selon la revendication 13 ou 14, dans lequel le plasma est un plasma pauvre en plaquettes.

23. Dispositif selon la revendication 7, dans lequel la résine comprend des ions calcium.

24. Dispositif selon la revendication 7 ou procédé selon la revendication 14, dans lequel la résine exclut des composés ayant un poids moléculaire supérieur à 20 000.

25. Dispositif selon la revendication 7 ou procédé selon la revendication 14, dans lequel la résine exclut des composés ayant un poids moléculaire supérieur à 30 000.
